(19) **Europäisches Patentamt / European Patent Office / Office européen des brevets**

(11) **EP 4 628 891 A1**

(12) **EUROPEAN PATENT APPLICATION**
published in accordance with Art. 153(4) EPC

(43) Date of publication:
**08.10.2025 Bulletin 2025/41**

(21) Application number: **23897784.7**

(22) Date of filing: **28.11.2023**

(51) International Patent Classification (IPC):
**G01N 33/49** (2006.01)   **A61K 31/365** (2006.01)
**A61P 37/06** (2006.01)

(52) Cooperative Patent Classification (CPC):
**A61K 31/365; A61P 37/06; G01N 33/49**

(86) International application number:
**PCT/JP2023/042587**

(87) International publication number:
**WO 2024/117136 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2022 JP 2022190246**

(71) Applicants:
• **Jichi Medical University**
**Tokyo 102-0093 (JP)**

• **SHIMADZU CORPORATION**
**Kyoto-shi, Kyoto 604-8511 (JP)**

(72) Inventors:
• **AIZAWA, Kenichi**
**Shimotsuke-shi, Tochigi 329-0498 (JP)**
• **IWAMI, Daiki**
**Shimotsuke-shi, Tochigi 329-0498 (JP)**
• **SAKUMA, Yasunaru**
**Shimotsuke-shi, Tochigi 329-0498 (JP)**

(74) Representative: **Müller-Boré & Partner**
**Patentanwälte PartG mbB**
**Friedenheimer Brücke 21**
**80639 München (DE)**

(54) **METHOD FOR QUANTIFYING ANALYTE CONTAINING MYCOPHENOLIC ACID AND ONE OR MORE OTHER IMMUNOSUPPRESSANTS IN TARGET BLOOD SAMPLE**

(57) The present invention provides a means for quantitatively analyzing an analyte comprising mycophenolic acid and one or more other immunosuppressants, using a whole blood fraction, without separating a blood sample. One aspect of the present invention relates to a method for quantifying an analyte comprising mycophenolic acid and one or more other immunosuppressants in a blood sample of a subject, wherein the method comprises: an analyte concentration-measuring step of measuring a concentration of the analyte comprising mycophenolic acid and one or more other immunosuppressants in a whole blood fraction of the blood sample; a hematocrit value-obtaining step of obtaining a hematocrit value of the blood sample of the subject; and a mycophenolic acid concentration-converting step of converting the concentration of mycophenolic acid in the whole blood fraction of the blood sample to a concentration of mycophenolic acid in a plasma fraction of the blood sample, based on the hematocrit value.

Figure 1

**Description**

TECHNICAL FIELD

The present invention relates to a method for quantifying an analyte comprising mycophenolic acid and one or more other immunosuppressants in a blood sample of a subject.

BACKGROUND ART

In transplantation therapy, the postoperative management of immunosuppressants is important for improving transplant outcomes and long-term prognosis. In organ transplant patients, multiple immunosuppressants are generally used to control the engraftment of transplanted organs. Examples of such an immunosuppressant may include mycophenolate mofetil (hereinafter also referred to as "mycophenolic acid"), tacrolimus, cyclosporine A, and everolimus.

In order to manage these immunosuppressants, therapeutic drug monitoring (TDM) is usually performed to measure and quantify the concentration of an immunosuppressant in the blood sample of an organ transplant patient. In the TDM, the concentration of an immunosuppressant in a specific blood sample, such as a whole blood fraction or a plasma fraction, is usually measured using a means such as immunoassay or mass spectrometry, depending on the type of the immunosuppressant as a target. For example, in the case of mycophenolic acid, the concentration of mycophenolic acid in a plasma fraction is usually measured, whereas in the case of tacrolimus, the concentration of tacrolimus in a whole blood fraction is usually measured.

SUMMARY OF INVENTION

TECHNICAL PROBLEM

Among the immunosuppressants commonly used in organ transplant patients, there are not only immunosuppressants whose concentrations are generally measured in the plasma fraction, such as mycophenolic acid, but there are also some immunosuppressants whose concentrations are typically measured in the whole blood fraction, such as tacrolimus, cyclosporine A, and everolimus. For this reason, in the TDM of immunosuppressants in blood samples from patients, including organ transplant patients, who have been administered with these immunosuppressants, simultaneous analysis could not be carried out.

Of the immunosuppressants commonly used in organ transplant patients, mycophenolic acid has a low correlation between trough values and pharmacological effects. Thus, it is recommended to determine the area under the blood concentration-time curve (AUC) value in the TDM of mycophenolic acid. However, in actual clinical practice, frequent blood sampling is highly invasive and often difficult. In addition, scheduled blood sampling is often difficult to implement in outpatient clinics. Therefore, patients would greatly benefit, if blood could be sampled at home using minimally invasive methods.

A technique of collecting blood by a patient his/herself at home using a minimally invasive means and quantifying a concentration of an analyte in the blood sample has been put to practical use, for example, as self-blood glucose level measurement. However, when such a technique of collecting and measuring blood by a patient his/herself is applied to the TDM of an immunosuppressant, for which a plasma fraction of a blood sample is generally used in measuring the blood concentration, like mycophenolic acid, there has been a problem that it is difficult to separate a blood sample and obtain a plasma fraction at the patient's home. Moreover, when the blood is collected by a patient his/herself according to a minimally invasive means, there has been a problem that the amount of blood collected is usually small.

Therefore, it is an object of the present invention to provide a means for quantitatively analyzing an analyte comprising mycophenolic acid and one or more other immunosuppressants, using a whole blood fraction, without separating a blood sample.

SOLUTION TO PROBLEM

The present inventors have conducted various studies regarding a means for achieving the above-described object. The present inventors have found that, in quantification of mycophenolic acid in a blood sample of a subject, a concentration of mycophenolic acid in a whole blood fraction of the blood sample can be converted with high accuracy to a concentration of mycophenolic acid in a plasma fraction of the blood sample, based on a hematocrit value of the blood sample. The present inventors have completed the present invention based on the above-described findings.

Specifically, the present invention includes the following embodiments.

(Embodiment 1)

A method for quantifying an analyte comprising mycophenolic acid and one or more other immunosuppressants in a blood sample of a subject, wherein the method comprises:

an analyte concentration-measuring step of measuring a concentration of the analyte comprising mycophenolic acid and one or more other immunosuppressants in a whole blood fraction of the blood sample;
a hematocrit value-obtaining step of obtaining a hematocrit value of the blood sample of the subject; and
a mycophenolic acid concentration-converting step of converting the concentration of mycophenolic acid in the whole blood fraction of the blood sample to a concentration of mycophenolic acid in a plasma fraction of the blood sample, based on the hematocrit value.

(Embodiment 2)

The method according to Embodiment 1, wherein the subject is an organ transplant patient.

(Embodiment 3)

The method according to Embodiment 1 or 2, wherein the one or more other immunosuppressants are selected from the group consisting of tacrolimus, cyclosporine A and everolimus.

(Embodiment 4)

The method according to any one of Embodiments 1 to 3, wherein the hematocrit value-obtaining step comprises measuring the hematocrit value of the blood sample of the subject.

(Embodiment 5)

The method according to any one of Embodiments 1 to 4, wherein the mycophenolic acid concentration-converting step is carried out based on the following equation:

$$A_p = A_w \times 100/(100\text{-}Ht)$$

wherein

$A_p$ is a converted value of the concentration of mycophenolic acid in the plasma fraction of the blood sample,
$A_w$ is the concentration of mycophenolic acid in the whole blood fraction, and
Ht is the hematocrit value of the blood sample.

(Embodiment 6)

The method according to any one of Embodiments 1 to 5, wherein the analyte concentration-measuring step is carried out by liquid chromatography mass spectrometry (LC-MS).

ADVANTAGEOUS EFFECTS OF INVENTION

According to the present invention, it becomes possible to provide a means for quantitatively analyzing an analyte comprising mycophenolic acid and one or more other immunosuppressants, using a whole blood fraction, without separating a blood sample. In addition, according to the present invention, it becomes possible to quantitatively analyzing an analyte in a trace amount of blood sample.

The present description includes the contents as disclosed in the description and/or drawings of Japanese Patent Application No. 2022-190246, which is a priority document of the present application.

BRIEF DESCRIPTION OF DRAWINGS

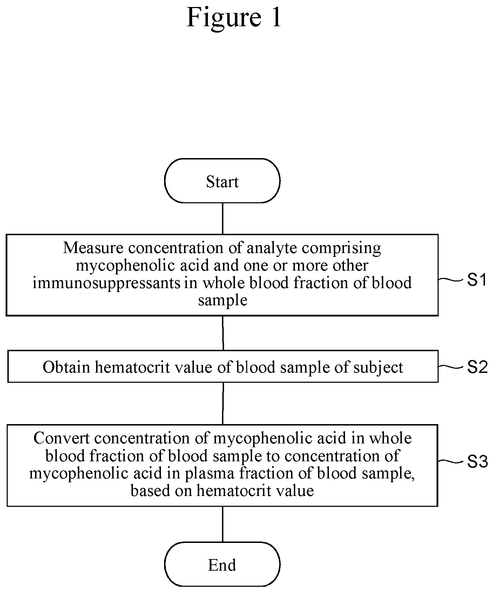

[Figure 1] Figure 1 is a flow chart showing individual steps of a method for quantifying an analyte comprising mycophenolic acid and one or more other immunosuppressants in a blood sample of a subject according to one aspect of the present invention.

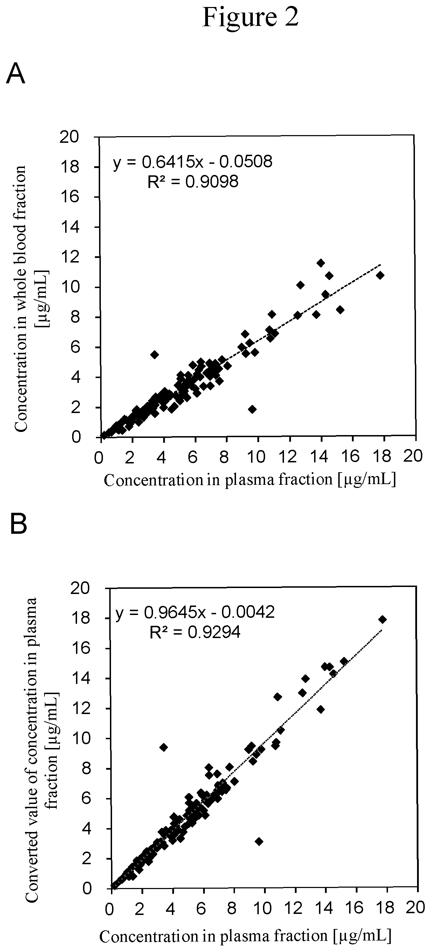

[Figure 2] Figure 2 is a graph showing the results of the measurement and conversion of the concentration of mycophenolic acid in blood samples from kidney transplant patients in Test I.
Figure 2A is a graph showing the correlation between the concentration of mycophenolic acid in the plasma fraction of each blood sample from kidney transplant patients and the concentration of mycophenolic acid in the whole blood fraction of each blood sample from the same patients. The horizontal axis is the concentration ($\mu$g/mL) of mycophenolic acid in the plasma fraction, and the longitudinal axis is the concentration ($\mu$g/mL) of mycophenolic acid in the whole blood fraction. Figure 2B is a graph showing the correlation between the concentration of mycophenolic acid in the plasma fraction of each blood sample from kidney transplant patients, and the converted value of the concentration of mycophenolic acid in the plasma fraction obtained by converting the concentration of mycophenolic acid in the whole blood fraction of each blood sample from the same patients. The horizontal axis is the concentration ($\mu$g/mL) of mycophenolic acid in the plasma fraction, and the longitudinal axis is the converted value ($\mu$g/mL) of the concentration of mycophenolic acid in the plasma fraction obtained by converting from the concentration of mycophenolic acid in the whole blood fraction.

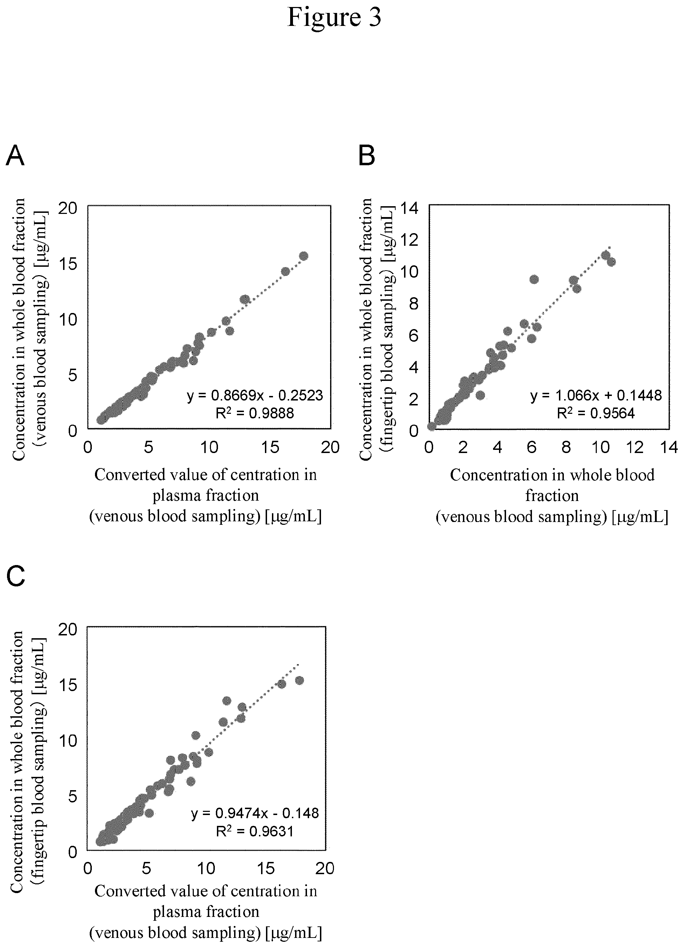

[Figure 3] Figure 3 is a graph showing the results of the measurement and conversion of the mycophenolic acid concentrations in blood samples collected from different sites of kidney transplant patients in Test II. Figure 3A is a graph showing the correlation between the concentration of mycophenolic acid in the whole blood fraction of a blood sample collected from the vein of kidney transplant patients, and the converted value of the concentration of mycophenolic acid in the plasma fraction, which was obtained by converting the concentration of mycophenolic acid in the whole blood fraction of a blood sample collected from the vein of kidney transplant patients. The horizontal axis is the converted value (μg/mL) of the concentration of mycophenolic acid in the plasma fraction obtained by converting the concentration of mycophenolic acid in the whole blood fraction of a blood sample collected from the vein of kidney transplant patients, and the longitudinal axis is the concentration (μg/mL) of mycophenolic acid in the whole blood fraction of a blood sample collected from the vein of kidney transplant patients. Figure 3B is a graph showing the correlation between the concentration of mycophenolic acid in the whole blood fraction of a blood sample collected from the fingertip of kidney transplant patients and the concentration of mycophenolic acid in the whole blood fraction of a blood sample collected from the vein of kidney transplant patients. The horizontal axis is the concentration (μg/mL) of mycophenolic acid in the whole blood fraction of a blood sample collected from the vein of kidney transplant patients, and the longitudinal axis is the concentration (μg/mL) of mycophenolic acid in the whole blood fraction of a blood sample collected from the fingertip of kidney transplant patients. Figure 3C is a graph showing the correlation between the concentration of mycophenolic acid in the whole blood fraction of a blood sample collected from the fingertip of kidney transplant patients, and the converted value of the concentration of mycophenolic acid in the plasma fraction, which was obtained by converting the concentration of mycophenolic acid in the whole blood fraction of a blood sample collected from the vein of kidney transplant patients. The horizontal axis is the converted value (μg/mL) of the concentration of mycophenolic acid in the plasma fraction obtained by converting the concentration of mycophenolic acid in the whole blood fraction of a blood sample collected from the vein of kidney transplant patients, and the longitudinal axis is the concentration of mycophenolic acid (μg/mL) in the whole blood fraction of a blood sample collected from the fingertip of kidney transplant patients.

DESCRIPTION OF EMBODIMENTS

< 1. Method for quantifying analyte comprising mycophenolic acid and one or more other immunosuppressants in blood sample >

Among the immunosuppressants commonly used in organ transplant patients, there are not only immunosuppressants whose concentrations are generally measured in the plasma fraction, such as mycophenolic acid, but there are also some immunosuppressants whose concentrations are typically measured in the whole blood fraction, such as tacrolimus, cyclosporine A, and everolimus. For this reason, in the TDM of immunosuppressants in blood samples from patients, including organ transplant patients, who have been administered with these immunosuppressants, simultaneous analysis could not be carried out.

In the TDM of immunosuppressants in organ transplant patients, it would be of great benefit to the patients, if blood could be collected at their own homes using minimally invasive means. However, when such a technique of collecting and measuring blood by a patient his/herself is applied to the TDM of an immunosuppressant, for which a plasma fraction of a blood sample is generally used in measuring the blood concentration, like mycophenolic acid, there has been a problem that it is difficult to separate a blood sample and obtain a plasma fraction at the patient's home. Moreover, when the blood is collected by a patient his/herself according to a minimally invasive means, there has been a problem that the amount of blood collected is usually small.

In response to these problems, the present inventors have found that, in quantification of mycophenolic acid in a blood sample of a subject, a concentration of mycophenolic acid in a whole blood fraction of the blood sample can be converted with high accuracy to a concentration of mycophenolic acid in a plasma fraction of the blood sample, based on a hematocrit value of the blood sample. Therefore, one aspect of the present invention relates to a method for quantifying an analyte comprising mycophenolic acid and one or more other immunosuppressants in a blood sample of a subject.

The subject, to whom the method of the present aspect is applied, may be a human or a non-human mammal. The method of the present aspect can be applied to patients, to whom mycophenolic acid is applied for the intended use of, for example, (1) the treatment of a refractory rejection reaction after kidney transplantation, (2) suppression of a rejection reaction in kidney, heart, liver, lung or pancreas transplantation, (3) the treatment of lupus nephritis, or (4) suppression of graft-versus-host disease in hematopoietic stem cell transplantation. It is known that there is no significant difference in the pharmacokinetics of mycophenolic acid in these patients. Therefore, the method of the present aspect can be applied to any of the patients exemplified above. The subject, to whom the method of the present aspect is applied, is preferably an organ transplant patient of any given solid organ, more preferably a patient with transplantation of one or more types of organs selected from the group consisting of kidney, liver, lung, heart, small intestine and pancreas, and further preferably an organ transplant patient of kidney or liver. In the above-exemplified subjects, in order to manage the amount of drugs used in the treatment, such as immunosuppressants, in the blood, TDM needs to be carried out. Therefore, by applying the method of the present aspect to the above-exemplified subjects, analyte comprising mycophenolic acid and one or more other immunosuppressants can be quantitatively analyzed using a whole blood fraction, without separating a blood sample.

The analyte comprising mycophenolic acid and one or more other immunosuppressants, to which the method of the present aspect is applied, is usually a drug used in the treatment of the above-exemplified subjects. For example, when the subject, to whom the method of the present aspect is applied, is an organ transplant patient, the analyte comprising mycophenolic acid and one or more other immunosuppressants is mycophenolic acid, as well as one or more other immunosuppressants selected from the group consisting of tacrolimus, cyclosporine A and everolimus. In the case of an organ transplant patient, it is important to carry out TDM to manage immunosuppressants after surgery, in order to improve transplant outcomes or long-term prognosis. For such an organ transplant patient, since it becomes important to suppress the immunity from both the cellular and antibody perspectives, usually, mycophenolic acid and one or more other immunosuppressants, as exemplified above are administered in combination. Therefore, by applying the method of the present aspect to the analyte comprising mycophenolic acid and one or more other immunosuppressants exemplified above, the analyte comprising mycophenolic acid and one or more other immunosuppressants can be quantitatively analyzed using the whole blood fraction, without separating the blood sample.

In the method of the present aspect, the blood sample of a subject can be obtained by a blood collection means commonly used in the present technical field. The used blood collection means can be appropriately selected by those skilled in the art, depending on the age, sex, and conditions (e.g., the severity of the symptoms, disease, and/or disorder of a subject) of a subject, to whom the method of the present aspect is applied, the blood collection site, the number of blood collections, the place of blood collection, and the amount of a blood sample or a fraction thereof used in individual steps described below. Examples of the blood collection site may include a vein in the arm and a fingertip. Examples of the blood collection means used may include a syringe and a blood collection tube, as well as an autologous blood collection kit for use in collection of a trace amount of blood. When a blood sample is obtained from a subject's fingertip using such an autologous blood collection kit for use in collection of a trace amount of blood, an analyte comprising mycophenolic acid and one or more other immunosuppressants can be quantitatively analyzed using the whole blood fraction of a trace amount (e.g., in the range of 0.01 to 0.1 mL) of blood sample.

A flow chart showing individual steps in one embodiment of the method of the present aspect is shown in Figure 1. As shown in Figure 1, the method of the present aspect comprises an analyte concentration-measuring step (S1), a hematocrit value-obtaining step (S2), and a mycophenolic acid concentration-converting step (S3). Hereinafter, each step will be described in detail.

[1-1. Analyte concentration-measuring step]

The method of the present aspect comprises an analyte concentration-measuring step (step S1) of measuring a concentration of an analyte comprising mycophenolic acid and one or more other immunosuppressants in a whole blood fraction of a blood sample.

The blood sample used in the present step can be obtained by the above-exemplified blood collection means.

The whole blood fraction of the blood sample used in the present step refers to the blood sample itself obtained by the above-exemplified blood collection means (i.e., an untreated blood sample).

In the present step, the means for measuring a concentration of an analyte comprising mycophenolic acid and one or more other immunosuppressants in a whole blood fraction of a blood sample may be, for example, immunoassay or an instrumental analysis method. The means for measuring a concentration of an analyte comprising mycophenolic acid and one or more other immunosuppressants in a whole blood fraction of a blood sample is preferably an instrumental analysis method, more preferably liquid chromatography, mass spectrometry or a combination thereof, further preferably liquid chromatography mass spectrometry (LC-MS), and particularly preferably liquid chromatography tandem mass spectrometry (LC-MS/MS or LC-MS/MS/MS, etc.). When the concentration of one or more analytes comprising mycophenolic acid is measured using an immunoassay, it is usually necessary to perform the measurement for every single analyte. In addition, several mL of the whole blood fraction is required for every single analyte. In contrast, when the concentration of an analyte comprising mycophenolic acid and one or more other immunosuppressants is measured using the above-exemplified instrumental analysis method, in general, the analytes can be analyzed simultaneously. Moreover, for a single measurement, only 1 mL or less of, for example, 0.01 to 0.1 mL of the whole blood fraction may be required. In particular, when LC-MS, preferably, liquid chromatography tandem mass spectrometry such as LC-MS/MS is used, the concentration of the analyte comprising mycophenolic acid and one or more other immunosuppressants can be measured even in a trace amount (for example, in the range of 0.01 to 0.1 mL) of whole blood fraction. Therefore, by measuring the concentration of the analyte comprising mycophenolic acid and one or more other immunosuppressants using the above-exemplified means, particularly, the above-exemplified instrumental analysis method, preferably, LC-MS, the analyte comprising mycophenolic acid and one or more other immunosuppressants can be quantitatively analyzed with high sensitivity, using a trace amount of whole blood fraction of a blood sample.

[1-2. Hematocrit value-obtaining step]

The method of the present aspect comprises a hematocrit value-obtaining step (step S2) of obtaining a hematocrit value of a blood sample of a subject.

In each aspect of the present invention, the hematocrit value refers to the percentage of the volume of the blood cell fraction relative to the total volume of a blood sample.

In the present step, the hematocrit value may be obtained from the measurement results of the hematocrit value of a blood sample of a subject that has been measured in advance, or may be obtained by measuring the hematocrit value of a blood sample of a subject when the method of the present aspect is carried out. By obtaining the hematocrit value from the measurement result of the hematocrit value of a blood sample of a subject that has been measured in advance, mycophenolic acid in the plasma fraction can be quantitatively analyzed without separating the blood sample.

[1-3. Mycophenolic acid concentration-converting step]

The method of the present aspect comprises a mycophenolic acid concentration-converting step (step S3) of converting the concentration of mycophenolic acid in the whole blood fraction of the blood sample to a concentration of mycophenolic acid in a plasma fraction of the blood sample, based on the hematocrit value.

The hematocrit value is the percentage of the volume of the blood cell fraction relative to the total volume of the blood sample. For this reason, if the analyte is substantially homogeneously present in a blood sample of a subject, the concentration of mycophenolic acid in the whole blood fraction of the blood sample can be converted to a concentration of mycophenolic acid in a plasma fraction of the blood sample, based on the hematocrit value. Therefore, by carrying out the present step, mycophenolic acid in a plasma fraction can be quantitatively analyzed using the whole blood fraction, without separating the blood sample.

The present step is preferably carried out based on the following equation:

$$A_p = A_w \times 100/(100\text{-}Ht)$$

wherein

$A_p$ is a converted value of the concentration of mycophenolic acid in the plasma fraction of the blood sample,
$A_w$ is the concentration of mycophenolic acid in the whole blood fraction, and
$Ht$ is the hematocrit value of the blood sample.

By converting the concentration of mycophenolic acid in the whole blood fraction of the blood sample to a concentration of mycophenolic acid in a plasma fraction of the blood sample based on the above equation, mycophenolic acid in the plasma fraction can be quantitatively analyzed using and the whole blood fraction. Therefore, according to the method of the present aspect, the analytes comprising mycophenolic acid and one or more other immunosuppressants can be simultaneously quantitatively analyzed using the whole blood fraction, without separating the blood sample.

< 2. Apparatus for quantifying analyte in blood sample >

Another aspect of the present invention relates to an apparatus for quantifying an analyte comprising mycophenolic acid and one or more other immunosuppressants in a blood sample of a subject.

The apparatus of the present aspect can be similarly applied to the subjects and analytes exemplified with regard to the method of one aspect of the present invention.

The apparatus of the present aspect includes an analyte concentration-measuring means, a hematocrit value-obtaining means, and a mycophenolic acid concentration-converting means. Each means will be described in detail below.

[2-1. Analyte concentration-measuring means]

The apparatus of the present aspect includes an analyte concentration-measuring means for measuring a concentration of an analyte comprising mycophenolic acid and one or more other immunosuppressants in a whole blood fraction of a blood sample.

The analyte concentration-measuring means usually has a sample introduction unit for introducing a whole blood fraction of a blood sample, and a concentration measurement unit for measuring a concentration of an analyte comprising mycophenolic acid and one or more other immunosuppressants in the whole blood fraction of a blood sample. The sample introduction unit may be configured separately from the concentration measurement unit, or may be configured integrally with the concentration measurement unit. The concentration measurement unit may be, for example, an immunoassay device or an instrumental analyzer. The concentration measurement unit is preferably an instrumental analyzer, more preferably a liquid chromatograph, a mass spectrometer or a combination thereof, further preferably a liquid chromatograph mass spectrometer (LC-MS), and particularly preferably a liquid chromatograph tandem mass spectrometer (LC-MS/MS or LC-MS/MS/MS, etc.). When the concentration measurement unit is the above-exemplified instrumental analyzer, the instrumental analyzer is usually configured integrally with the sample introduction unit, and one or more analytes comprising mycophenolic acid can be measured simultaneously. Moreover, for a single measurement, only 1 mL or less of, for example, 0.01 to 0.1 mL of the whole blood fraction may be required. In particular, when the concentration measurement unit is LC-MS, preferably, a liquid chromatography tandem mass spectrometer such as LC-MS/MS, the concentration of the analyte comprising mycophenolic acid and one or more other immunosuppressants can be measured even in a trace amount (for example, in the range of 0.01 to 0.1 mL) of a whole blood fraction. Therefore, by measuring the concentration of the analyte comprising mycophenolic acid and one or more other immunosuppressants using the above-exemplified sample introduction unit and concentration measurement unit, particularly, the above-exemplified instrumental analyzer, preferably, LC-MS, the analyte comprising mycophenolic acid and one or more other immunosuppressants can be quantitatively analyzed with high sensitivity, using the whole blood fraction of a trace amount of a blood sample.

[2-2. Hematocrit value-obtaining means]

The apparatus of the present aspect includes a hematocrit value-obtaining means for obtaining a hematocrit value of a blood sample of a subject.

In the hematocrit value-obtaining means, the hematocrit value may be obtained from measurement results of a hematocrit value of a blood sample of a subject that has been measured in advance, or may be obtained by measuring a hematocrit value of a blood sample of a subject when the apparatus of the present aspect is used. In the case of the former embodiment, the hematocrit value-obtaining means usually has a central control unit, a data storage unit, an input unit, and an output unit. In the data storage unit, there are stored measurement results of a hematocrit value of a blood sample of a subject that has been previously measured. In the case of the present embodiment, the hematocrit value-obtaining means is preferably in the form of a computer having a central processing device, a storage device such as a hard drive, an input device and an output device. In the case of the latter embodiment, the hematocrit value-obtaining means may be a hematocrit value measuring device that is commonly used in the present technical field. In the apparatus of the present aspect, by obtaining a hematocrit value from measurement results of a hematocrit value of a blood sample of a subject that has been previously measured, mycophenolic acid in the plasma fraction can be quantitatively analyzed without separating the blood sample.

[2-3. Mycophenolic acid concentration-converting means]

The apparatus of the present aspect includes a mycophenolic acid concentration-converting means for converting the concentration of mycophenolic acid in the whole blood fraction of the blood sample to a concentration of mycophenolic acid in the plasma fraction of the blood sample, based on the hematocrit value.

The mycophenolic acid concentration-converting means usually has a central control unit, an analysis processing unit, a data storage unit, an input unit and an output unit. In the data storage unit, in general, a program for performing conversion of the concentration of mycophenolic acid is stored. The mycophenolic acid concentration-converting means is preferably in the form of a computer having, for example, a central processing device, a storage device such as a hard drive, an input device and an output device. In the apparatus of the present aspect, by converting the concentration of mycophenolic acid in the whole blood fraction of the blood sample to a concentration of mycophenolic acid in the plasma fraction of the blood sample, an analyte comprising mycophenolic acid and one or more other substances can be quantitatively analyzed using the whole blood fraction, without separating the blood sample.

EXAMPLES

Hereinafter, the present invention will be further specifically described using the following examples. However, these examples are not intended to limit the technical scope of the present invention.

< Test I: Measurement and conversion of immunosuppressant concentrations in blood samples of kidney transplant patients >

One hundred and thirty-four blood samples (2 mL) were obtained from a group of patients after kidney transplantation. A portion was fractionated from each blood sample as a whole blood fraction. The remaining portion was centrifuged to prepare a plasma fraction. The concentrations of mycophenolic acids (MPA) in the whole blood fraction (10 $\mu$L) and the plasma fraction (10 $\mu$L) of each blood sample were each measured by liquid chromatography tandem mass spectrometry (LC-MS/MS). In addition, the hematocrit value of each blood sample was also measured. Based on the hematocrit value of each blood sample and the following equation, the concentration of mycophenolic acid in the whole blood fraction of the blood sample was converted to the concentration of mycophenolic acid in the plasma fraction.

$$A(MPA)_p = A(MPA)_w \times 100/(100\text{-}Ht)$$

wherein

$A(MPA)_p$ is the converted value of the concentration of mycophenolic acid in the plasma fraction of the blood sample,
$A(MPA)_w$ is the concentration of mycophenolic acid in the whole blood fraction, and
Ht is the hematocrit value of the blood sample.

The results of the measurement and conversion of the concentrations of mycophenolic acids in blood samples from kidney transplant patients are shown in Figure 2. In Figure 2, Figure 2A is a graph showing the correlation between the concentration of mycophenolic acid in the plasma fraction of each blood sample from kidney transplant patients and the concentration of mycophenolic acid in the whole blood fraction of each blood sample from the same patients. The horizontal axis is the concentration ($\mu$g/mL) of mycophenolic acid in the plasma fraction, and the longitudinal axis is the concentration ($\mu$g/mL) of mycophenolic acid in the whole blood fraction. Figure 2B is a graph showing the correlation between the concentration of mycophenolic acid in the plasma fraction of each blood sample from kidney transplant patients, and the converted value of the concentration of mycophenolic acid in the plasma fraction obtained by converting the concentration of mycophenolic acid in the whole blood fraction of each blood sample from the same patients. The horizontal axis is the concentration ($\mu$g/mL) of mycophenolic acid in the plasma fraction, and the longitudinal axis is the converted value ($\mu$g/mL) of the concentration of mycophenolic acid in the plasma fraction obtained by converting from the concentration of mycophenolic acid in the whole blood fraction.

As shown in Figure 2A, a certain correlation was confirmed between the concentration of mycophenolic acid in the plasma fraction of the blood sample and the concentration of mycophenolic acid in the whole blood fraction of the blood sample, which were both from the same patient. Herein, when the concentration of mycophenolic acid in the whole blood fraction of each blood sample was converted to the concentration of mycophenolic acid in the plasma fraction based on the hematocrit value and each blood sample and the above-described equation, the converted value was well matched with the actual value of mycophenolic acid in the plasma fraction of the blood sample from the same patient (Figure 2B).

< Test II: Comparison regarding measurement and conversion of immunosuppressant concentrations in blood samples collected from different sites of kidney transplant patients

Seventy-two blood samples (2 mL) were obtained from the arm veins of a group of patients after kidney transplantation. Seventy-two blood samples (5.6 $\mu$L) were obtained from the fingertips of the same group of patients using an autologous blood collection kit for use in collection of a trace amount of blood (MSW2). The concentration of MPA in the whole blood fraction (5.6 $\mu$L) of each blood sample collected from vein and fingertip was measured by LC-MS/MS. In addition, the hematocrit value of each blood sample was also measured. Based on the hematocrit value of each blood sample and the equation used in Test I, the concentration of mycophenolic acid in the whole blood fraction of each blood sample was converted to the concentration of mycophenolic acid in the plasma fraction. However, one case, in which the concentration of mycophenolic acid in the whole blood fraction of the blood sample collected from the fingertip was significantly deviated from the values calculated in the previous tests was excluded from the statistical processing of the concentration of mycophenolic acid in the whole blood fraction of the blood sample collected from the fingertip.

The results of the measurement and conversion of the mycophenolic acid concentrations in blood samples collected from different sites of kidney transplant patients are shown in Figure 3. In Figure 3, Figure 3A is a graph showing the correlation between the concentration of mycophenolic acid in the whole blood fraction of a blood sample collected from the vein of kidney transplant patients and the converted value of the concentration of mycophenolic acid in the plasma fraction obtained by converting the concentration of mycophenolic acid in the whole blood fraction of a blood sample collected from the vein of kidney transplant patients. The horizontal axis is the converted value ($\mu$g/mL) of the concentration of mycophenolic acid in the plasma fraction obtained by converting the concentration of mycophenolic acid in the whole blood fraction of a blood sample collected from the vein of kidney transplant patients, and the longitudinal axis is the concentration ($\mu$g/mL) of mycophenolic acid in the whole blood fraction of a blood sample collected from the vein of kidney transplant patients. Figure 3B is a graph showing the correlation between the concentration of mycophenolic acid in the whole blood fraction of a blood sample collected from the fingertip of kidney transplant patients and the concentration of mycophenolic acid in the whole blood fraction of a blood sample collected from the vein of kidney transplant patients. The horizontal axis is the concentration ($\mu$g/mL) of mycophenolic acid in the whole blood fraction of a blood sample collected from the vein of kidney transplant patients, and the longitudinal axis is the concentration ($\mu$g/mL) of mycophenolic acid in the whole blood fraction of a blood sample collected from the fingertip of kidney transplant patients. Figure 3C is a graph showing the correlation between the concentration of mycophenolic acid in the whole blood fraction of a blood sample collected from the fingertip of kidney transplant patients and the converted value of the concentration of mycophenolic acid in the plasma fraction obtained by converting the concentration of mycophenolic acid in the whole blood fraction of a blood sample collected from the vein of kidney transplant patients. The horizontal axis is the converted value ($\mu$g/mL) of the concentration of mycophenolic acid in the plasma fraction obtained by converting the concentration of mycophenolic acid in the whole blood fraction of a blood sample collected from the vein of kidney transplant patients, and the longitudinal axis is the concentration of mycophenolic acid ($\mu$g/mL) in the whole blood fraction of a blood sample collected from the fingertip of kidney transplant patients.

As shown in Figure3 A, the converted value of the concentration of mycophenolic acid in the plasma fraction obtained by converting the concentration of mycophenolic acid in the whole blood fraction of a blood sample collected from the vein of a kidney transplant patient was well matched with the actual value of the concentration of mycophenolic acid in the whole blood fraction of the blood sample collected from the vein of the same patient. Moreover, as shown in Figure3B, the actual value of the concentration of mycophenolic acid in the whole blood fraction of a blood sample collected from the fingertip of a kidney transplant patient was well matched with the actual value of the concentration of mycophenolic acid in the whole blood fraction of the blood sample collected from the vein of the same patient. Furthermore, as shown in Figure3C, the actual value of the concentration of mycophenolic acid in the whole blood fraction of a blood sample collected from the fingertip of a kidney transplant patient was also well matched with the converted value of the concentration of mycophenolic acid in the plasma fraction, which was obtained by converting the concentration of mycophenolic acid in the whole blood fraction of the blood sample collected from the vein of the same patient. From these results, it became clear that, even in a case where blood samples collected from different sites are used, mycophenolic acid can be quantitatively analyzed using the whole blood fraction by applying the method of the present invention.

Regarding mycophenolic acid, it has been thought that, after mycophenolic acid has been absorbed into the body, most of it is present in whole blood, especially in the plasma fraction. However, there have been no particular data to support this assumption so far. Moreover, also tacrolimus, which is used as an immunosuppressant, is mostly present in erythrocytes, which are particularly present in the blood cell fraction in whole blood. From this fact, it had been thought that mycophenolic acid may also be present in the blood cell fraction. The present inventors have discovered for the first time that most of mycophenolic acid is present in the plasma fraction. Thus, based on the fact that most of mycophenolic acid is present in whole blood, especially in the plasma fraction after it has been absorbed into the body, the present inventors have conceived of an idea that when the concentration of mycophenolic acid in whole blood is converted to the concentration of mycophenolic acid in the plasma fraction, a value obtained by calculating the percentage of the volume of the plasma fraction to the total volume of the blood sample based on the hematocrit value that is the percentage of the volume of the blood cell fraction to the total volume of the blood sample, is used, thereby completing the present invention.

It is to be noted that the present invention is not limited to the above-described examples, but it includes various modified examples. For example, the above-described examples have been described in detail to make the present invention easier to understand, and the present invention is not necessarily limited to those having all of the configurations described. In addition, an addition, deletion, and/or substitution of other configurations is possible for a part of the configuration of each example.

All publications, patents and patent applications cited in the present description are incorporated herein by reference in their entirety.

## Claims

1. A method for quantifying an analyte comprising mycophenolic acid and one or more other immunosuppressants in a blood sample of a subject, wherein the method comprises:

    an analyte concentration-measuring step of measuring a concentration of the analyte comprising mycophenolic acid and one or more other immunosuppressants in a whole blood fraction of the blood sample;
    a hematocrit value-obtaining step of obtaining a hematocrit value of the blood sample of the subject; and
    a mycophenolic acid concentration-converting step of converting the concentration of mycophenolic acid in the whole blood fraction of the blood sample to a concentration of mycophenolic acid in a plasma fraction of the blood sample, based on the hematocrit value.

2. The method according to claim 1, wherein the subject is an organ transplant patient.

3. The method according to claim 1, wherein the one or more other immunosuppressants are selected from the group consisting of tacrolimus, cyclosporine A and everolimus.

4. The method according to claim 1, wherein the hematocrit value-obtaining step comprises measuring the hematocrit value of the blood sample of the subject.

5. The method according to claim 1, wherein the mycophenolic acid concentration-converting step is carried out based on the following equation:

$$A_p = A_w \times 100/(100-Ht)$$

wherein

    $A_p$ is a converted value of the concentration of mycophenolic acid in the plasma fraction of the blood sample,
    $A_w$ is the concentration of mycophenolic acid in the whole blood fraction, and
    Ht is the hematocrit value of the blood sample.

6. The method according to claim 1, wherein the analyte concentration-measuring step is carried out by liquid chromatography mass spectrometry (LC-MS).

# Figure 1

```
                    ┌─────────────┐
                    │    Start    │
                    └──────┬──────┘
                           │
    ┌──────────────────────┴──────────────────────┐
    │  Measure concentration of analyte comprising │
    │   mycophenolic acid and one or more other    │ ─ S1
    │ immunosuppressants in whole blood fraction of│
    │                blood sample                  │
    └──────────────────────┬──────────────────────┘
                           │
    ┌──────────────────────┴──────────────────────┐
    │ Obtain hematocrit value of blood sample of   │ ─ S2
    │                  subject                     │
    └──────────────────────┬──────────────────────┘
                           │
    ┌──────────────────────┴──────────────────────┐
    │ Convert concentration of mycophenolic acid in│
    │ whole blood fraction of blood sample to      │ ─ S3
    │ concentration of mycophenolic acid in plasma │
    │ fraction of blood sample, based on hematocrit│
    │                   value                      │
    └──────────────────────┬──────────────────────┘
                           │
                    ┌──────┴──────┐
                    │    End      │
                    └─────────────┘
```

# Figure 2

A

B

# Figure 3

A

Concentration in whole blood fraction (venous blood sampling) [μg/mL]

$y = 0.8669x - 0.2523$
$R^2 = 0.9888$

Converted value of centration in plasma fraction
(venous blood sampling) [μg/mL]

B

Concentration in whole blood fraction (fingertip blood sampling) [μg/mL]

$y = 1.066x + 0.1448$
$R^2 = 0.9564$

Concentration in whole blood fraction
(venous blood sampling) [μg/mL]

C

Concentration in whole blood fraction (fingertip blood sampling) [μg/mL]

$y = 0.9474x - 0.148$
$R^2 = 0.9631$

Converted value of centration in plasma fraction
(venous blood sampling) [μg/mL]

## INTERNATIONAL SEARCH REPORT

| International application No. |
|---|
| **PCT/JP2023/042587** |

### A. CLASSIFICATION OF SUBJECT MATTER

*G01N 33/49*(2006.01)i; *A61K 31/365*(2006.01)i; *A61P 37/06*(2006.01)i
FI:  G01N33/49 Z; G01N33/49 B; A61K31/365; A61P37/06

According to International Patent Classification (IPC) or to both national classification and IPC

### B. FIELDS SEARCHED

Minimum documentation searched (classification system followed by classification symbols)

G01N33/49; A61K31/365; A61P37/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | JP 2013-036959 A (TOYOBO CO., LTD.) 21 February 2013 (2013-02-21) <br> see entire text, all drawings, particularly, claims, paragraphs [0004]-[0005], [0033], etc. | 1-6 |
| Y | JP 2021-192009 A (A&T CORPORATION) 16 December 2021 (2021-12-16) <br> see entire text, all drawings, particularly, claims, paragraphs [0003], [0021], etc. | 1-6 |
| Y | US 2011/0281369 A1 (AKHLAGHI, Fatemeh) 17 November 2011 (2011-11-17) <br> see entire text, all drawings, particularly, abstract, claims, paragraphs [0002]-[0004], [0028]-[0030], [0042]-[0045], table 2, etc. | 1-6 |
| Y | CN 1621835 A (FUDAN UNIVERSITY) 01 June 2005 (2005-06-01) <br> see entire text, all drawings, particularly, claims, page 1, lines 4-20, etc. | 1-6 |
| A | JP 2008-524239 A (ELAN PHARMA INTERNATIONAL LTD.) 10 July 2008 (2008-07-10) <br> see entire text, all drawings, particularly, paragraphs [0015], [0071], etc. | 1-6 |

☑ Further documents are listed in the continuation of Box C.    ☑ See patent family annex.

| | |
|---|---|
| \* Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **31 January 2024** | **13 February 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**

| International application No. |
| --- |
| **PCT/JP2023/042587** |

**C.    DOCUMENTS CONSIDERED TO BE RELEVANT**

| Category* | Citation of document, with indication, where appropriate, of the relevant passages | Relevant to claim No. |
| --- | --- | --- |
| A | TSUNODA, S. M. and 1 other, The Use of Therapeutic Drug Monitoring to Optimise Immunosuppressive Therapy, Clin Pharmacokinet, 1996, vol. 30, no. 2, pages 107-140, doi: 10.2165/00003088-199630020-00003, PMID: 8906895<br>see entire text, particularly, page 121, right column, third paragraph to page 126, left column, first paragraph, page 130, right column, second paragraph to page 131, right column, third paragraph, etc. | 1-6 |

Form PCT/ISA/210 (second sheet) (July 2022)

## INTERNATIONAL SEARCH REPORT
### Information on patent family members

International application No.

**PCT/JP2023/042587**

| Patent document cited in search report | | | Publication date (day/month/year) | Patent family member(s) | Publication date (day/month/year) |
|---|---|---|---|---|---|
| JP | 2013-036959 | A | 21 February 2013 | (Family: none) | |
| JP | 2021-192009 | A | 16 December 2021 | US 2023/0228734 A1 see entire text, all drawings, particularly, claims, paragraphs [0003], [0023], etc. WO 2021/246096 A1 | |
| US | 2011/0281369 | A1 | 17 November 2011 | US 2008/0318322 A1 see entire text, all drawings, particularly, abstract, claims, paragraphs [0002]-[0004], [0028]-[0030], [0042]-[0045], table 2, etc. WO 2007/090081 A2 | |
| CN | 1621835 | A | 01 June 2005 | (Family: none) | |
| JP | 2008-524239 | A | 10 July 2008 | US 2006/0159766 A1 see entire text, all drawings, particularly, paragraphs [0093]-[0094], etc. US 2008/0152720 A1 US 2009/0252806 A1 WO 2006/066063 A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022190246 A